# FASCICULE DE BREVET EUROPEEN

(11) **EP 4 132 898 B1**
(45) Date de publication et mention de la délivrance du brevet: **23.04.2025**
(21) Numéro de dépôt: 21717151.1
(22) Date de dépôt: 23.03.2021
(51) Int. Cl.: C07C 57/04, C07C 51/44

(54) **PROCEDE DE PURIFICATION D'ACIDE (METH)ACRYLIQUE**
VERFAHREN ZUR REINIGUNG VON (METH)ACRYLSÄURE
METHOD FOR PURIFYING (METH)ACRYLIC ACID

(30) Priorité: 06.04.2020 FR 2003402
(43) Date de publication de la demande: 15.02.2023
(73) Titulaire: ARKEMA FRANCE, 92800 Puteaux - La Défense (FR)
(72) Inventeur: TRETJAK, Serge, 57501 SAINT AVOLD CEDEX (FR); HUVE, Aurélien, 57501 SAINT AVOLD CEDEX (FR)
(74) Mandataire: Arkema Patent
(86) Numéro de dépôt international: PCT/FR2021/050490
(87) Numéro de publication internationale: WO 2021/205090

(56) Documents cités:
- WO-A1-2015/126704
- WO-A1-2016/142608

## Description

### DOMAINE DE L'INVENTION

La présente invention concerne un procédé de purification amélioré d'acide (méth)acrylique, mis en œuvre en l'absence de solvant organique et en l'absence d'un traitement chimique des aldéhydes.

### ARRIERE-PLAN TECHNIQUE

Le procédé de synthèse d'acide acrylique exploité à grande échelle industrielle, met en œuvre une réaction d'oxydation catalytique du propylène en présence d'oxygène. Cette réaction est conduite généralement en phase gazeuse, et le plus souvent en deux étapes : la première étape réalise l'oxydation sensiblement quantitative du propylène en un mélange riche en acroléine, puis, la deuxième étape réalise l'oxydation sélective de l'acroléine en acide acrylique.

Le mélange gazeux issu de la deuxième étape est constitué, en dehors de l'acide acrylique, de composés non transformés issus des réactifs mis enjeu ou d'impuretés générées lors de l'une au moins des deux étapes de réaction, à savoir :
- de composés légers incondensables dans les conditions de température et de pression habituellement mises en œuvre, soit essentiellement : propylène, propane, azote, oxygène non converti, monoxyde et dioxyde de carbone formés en faible quantité par oxydation ultime;
- de composés légers condensables, soit essentiellement : eau, des aldéhydes légers comme acroléine non converti, formaldéhyde, glyoxal et acétaldéhyde, acide formique, acide acétique, acide propénoïque ;
- de composés lourds : furfuraldéhyde, benzaldéhyde, acide et anhydride maléique, acide benzoïque, acide 2-buténoïque, phénol, protoanémonine.

La complexité du mélange gazeux obtenu dans ce procédé nécessite un ensemble d'opérations pour récupérer l'acide acrylique contenu dans cet effluent gazeux et le transformer en un grade d'acide acrylique technique ou glacial compatible avec son utilisation finale, par exemple la synthèse d'esters acryliques ou la production de polymères d'acide acrylique et/ou d'esters acryliques.

Les documents EP 2 066 613, WO 2015/126704 et WO 2008/033687, basés sur une technologie « sans solvant », décrivent un procédé de récupération d'acide acrylique sans utiliser d'eau extérieure, ni de solvant azéotropique. Ce procédé met en œuvre deux colonnes de distillation pour purifier le mélange réactionnel gazeux refroidi : a) une colonne de déshydratation, b) une colonne de finition alimentée par une partie du flux de pied de la colonne de déshydratation. Lors de l'étape de purification/finition, on récupère un flux d'acide acrylique purifié sous forme de liquide ou de vapeur, par soutirage latéral de la colonne de finition. L'acide acrylique technique obtenu est généralement de pureté supérieure à 98,5% voire supérieure à 99,5 % (teneurs massiques). Il contient moins de 0,3% en eau, moins de 0,075% d'acide acétique et surtout des composés lourds tels que des aldéhydes et de la protoanémonine.

Cet acide acrylique technique peut être utilisé, sans autre purification, pour produire des esters. Cette qualité « technique » ne va cependant pas être suffisante lorsque cet acide acrylique est destiné à la fabrication de polymères. Dans ce cas il sera nécessaire d'utiliser de l'acide acrylique dit glacial. Il est en effet particulièrement important d'éliminer dans l'AA technique certaines impuretés jusqu'à des teneurs extrêmement faibles. Il s'agit notamment de certains aldéhydes, comme le furfuraldéhyde (ou furfural), le benzaldéhyde ou l'acroléine, ou d'autres impuretés comme la protoanémonine, composés générés au cours de la synthèse de l'acide acrylique, ou encore les inhibiteurs de polymérisation non phénoliques, comme la phénothiazine introduite lors de la synthèse de l'acide acrylique. Ces composés ont un effet important sur la réactivité de l'AA glacial lorsqu'il est engagé dans une réaction de polymérisation visant à produire un polymère de haute masse moléculaire, en retardant ou en inhibant cette réaction.

L'acide acrylique glacial est destiné ensuite à la mise en œuvre de procédés de polymérisation, soit de l'acide acrylique, soit de ses dérivés esters ou amide, procédés qui sont conduits sous différentes formes, en masse, en solution, en suspension, ou en émulsion. Ces polymères sont utilisés tels quels ou en tant que copolymères dans des domaines aussi variés que l'hygiène (par exemple dans la production de super absorbants), les détergents, les peintures, les vernis, les adhésifs, le papier, les textiles ou le cuir.

Pour obtenir de l'acide acrylique glacial à partir d'acide acrylique technique, les documents EP2066613 ou WO 2008/033687 indiquent que ce dernier peut être soumis à un traitement complémentaire par cristallisation fractionnée, comme décrit dans le brevet US5504247 de Sulzer, ou dans le document WO 2011/010035 de la Société Demanderesse pour la production d'acide acrylique de grade polymère d'origine renouvelable.

Par ailleurs, un procédé de fabrication d'AA glacial par distillation peut être avantageux grâce à ses possibilités d'intégration énergétique quand on peut récupérer les calories présentes dans l'énergie excédentaire fournie par l'étape exothermique de réaction (sous forme de vapeur), ce qui ne peut pas être réalisé simplement lorsque la purification est réalisée par cristallisation (celle-ci nécessitant des frigories, et donc une consommation électrique). On peut ainsi obtenir de l'acide acrylique glacial par une opération de distillation combinée à un traitement chimique permettant d'éliminer les aldéhydes. Parmi les réactifs utilisables, on peut employer des amines, et plus particulièrement les composés de la famille des hydrazines, comme décrit dans les brevets US 3,725,208 et US 7,393,976.

Dans le document WO 2017/060583, l'étape de traitement des aldéhydes à l'aide d'un agent chimique est réalisée dans une section de purification comprenant une colonne de déshydratation, et une colonne de finition, de préférence à l'intérieur de ladite section de purification, ou en alternative dans une section de purification complémentaire par distillation avec une ou deux colonnes de distillation, et permettant de conduire à une qualité d'acide acrylique glacial.

Le document WO 2018/185423 décrit la mise en œuvre d'une colonne à paroi séparatrice comme colonne de purification/finition dans un procédé de récupération d'acide acrylique utilisant deux colonnes de distillation en l'absence de solvant organique externe. La configuration particulière de la colonne à paroi séparatrice, c'est-à-dire lorsque la paroi séparatrice est jointive avec le dôme supérieur de la colonne en partie haute, et non jointive avec le fond de la colonne en partie basse, permet d'améliorer le bilan énergétique du procédé tout en améliorant la qualité technique de l'acide acrylique récupéré. L'acide acrylique technique extrait en tête de la colonne à paroi séparatrice peut être soumis à un traitement complémentaire par cristallisation fractionnée, ou par distillation éventuellement en présence d'un composé réagissant avec les aldéhydes résiduels. Dans certaines conditions opératoires particulières, la qualité de l'acide acrylique obtenue est celle d'un acide acrylique glacial.

La mise en œuvre d'une colonne de distillation à paroi séparatrice reste complexe et la purification décrite dans ce document ne peut pas être adaptée, sans modification significative, aux procédés conventionnels de récupération d'acide acrylique utilisant un solvant organique externe.

Le document WO 2016/142608 décrit un procédé de récupération d'acide (méth)acrylique sans utiliser de solvant azéotropique, à partir d'un mélange réactionnel gazeux comprenant de l'acide (méth)acrylique obtenu par oxydation en phase gazeuse d'un précurseur de l'acide (méth)acrylique, comprenant au moins les étapes suivantes :
i) on soumet le mélange réactionnel gazeux à une déshydratation sans utiliser de solvant azéotropique dans une première colonne dite colonne de déshydratation, conduisant à un flux de tête dont une partie au moins est condensée et renvoyée à la colonne de déshydratation sous forme de reflux, et à un flux de pied ;
ii) on soumet au moins en partie le flux de pied de colonne de déshydratation à une distillation à une pression inférieure à la pression atmosphérique dans une seconde colonne dite colonne de finition, conduisant à un flux de tête, et à un flux de pied contenant des composés lourds ;
iii) on récupère un flux d'acide (méth)acrylique par soutirage latéral de la colonne de finition, et/ou en pied de la colonne de finition ;
ledit procédé étant caractérisé en ce que le flux de tête de la colonne de finition est au moins en partie soumis à un système de condensation à pompe à vide sèche, formant un condensat qui est renvoyé dans la colonne de déshydratation, et un effluent gazeux ultime.

Ce document indique qu'un flux d'acide (méth)acrylique 16 est récupéré par soutirage latéral à partir de la colonne de finition (étape iii), à un niveau latéral situé de préférence en-dessous de l'alimentation de ladite colonne, et que ledit flux 16 peut encore être soumis à une purification par distillation, éventuellement couplée avec un traitement de cristallisation.

La demanderesse a décrit dans sa demande FR1903519 un procédé de purification d'un acide acrylique technique en l'absence de réactif chimique de traitement des aldéhydes, produisant un flux d'acide acrylique glacial qui est soutiré par une sortie latérale de l'unité de distillation, un flux comportant essentiellement des composés légers étant extrait en tête de l'unité de distillation, et un flux d'acide acrylique comportant des composés lourds étant récupéré en pied de l'unité de distillation.

Il existe maintenant le besoin de fournir un procédé de purification optimisé « sans solvant » et « sans agent de traitement chimique » permettant d'obtenir en même temps de l'acide acrylique glacial et de l'acide acrylique technique, en optimisant la consommation énergétique globale de l'ensemble du train de séparation ainsi que celles des ateliers utilisant ces acides acryliques, technique ou glacial. En adaptant le ratio de production acide acrylique glacial /acide acrylique technique, le procédé selon l'invention permet de s'accorder aux demandes du marché.

### RESUME DE L'INVENTION

La présente invention a pour objet un procédé de production en continu d'acide acrylique, en l'absence de solvant organique et en l'absence d'un traitement chimique des aldéhydes, et sans employer de colonne à paroi séparatrice, à partir d'un mélange réactionnel gazeux comprenant de l'acide acrylique obtenu par oxydation en phase gazeuse d'un précurseur de l'acide acrylique, ledit procédé mettant en œuvre trois colonnes de distillation et comprenant les étapes suivantes :
a) ledit mélange réactionnel gazeux est soumis à une déshydratation sans utiliser de solvant azéotropique, dans une première colonne de distillation dite colonne de déshydratation, conduisant à un flux de tête dont une partie au moins est condensée et renvoyée à la colonne de déshydratation sous forme de reflux, et à un flux de pied dont une partie au moins est renvoyée en reflux dans la partie inférieure de la colonne de déshydratation pour former une boucle de recirculation ;
b) le flux de pied de la colonne de déshydratation est envoyé au moins en partie dans une seconde colonne de distillation dite colonne de finition. La colonne de finition est alimentée sur le premier tiers haut de la colonne et on procède à la distillation des produits légers, l'élimination des produits lourds en pied de cette colonne, l'obtention d'acide acrylique de qualité technique en soutirage latéral en phase gazeuse placé dans le premier tiers inférieur de cette colonne. Les produits légers composés en partie de l'eau et de l'acide acétique sont extraits en tête de la section d'alimentation, puis recyclé, après condensation, au moins en partie dans la boucle de recirculation en pied de la colonne de déshydratation ou comme reflux liquide en tête de cette colonne dite de finition ;
c) l'acide acrylique technique extrait par le soutirage latéral en phase gazeuse alimente une troisième colonne de distillation qui permet d'obtenir de l'acide acrylique technique par soutirage latéral au tiers inférieur de cette colonne, et de l'acide acrylique glacial en tête de cette colonne ; une partie, au moins, de ce dernier, est condensée et renvoyée dans cette colonne sous forme de reflux. En outre, le produit résiduel issu de la distillation de l'acide acrylique glacial, récupéré en pied de l'unité de distillation, peut être avantageusement recyclé vers un atelier d'estérification fabriquant des esters acryliques en C1-C8, sans purification supplémentaire qui serait indispensable en cas d'utilisation d'un agent de traitement chimique des aldéhydes.

L'invention vise à produire de façon continue de l'acide acrylique technique ainsi que de l'acide glacial avec un coût de purification optimisé. Elle est basée sur la mise en œuvre dans des conditions particulières d'un procédé de purification impliquant un nombre réduit de colonnes de distillation et ne nécessitant pas de solvant organique externe, ni de traitement chimique destiné à réduire la teneur en aldéhydes, ni la mise en œuvre de colonne de distillation à paroi séparatrice.

Le procédé selon l'invention permet de produire simultanément, à l'issue de la dernière étape de distillation :
- un flux d'acide acrylique glacial ayant une pureté supérieure à 99,7% et des teneurs massiques en impuretés comme suit : teneur en furfural < 2 ppm ; teneur en aldéhydes totaux (furfural, benzaldéhyde, acroléine) < 10 ppm, de préférence < 4 ppm ; teneur en protoanémonine < 2 ppm, et
- un flux d'acide acrylique technique ayant de pureté supérieure à 99,5% massique et contenant moins de 0,3% en eau, moins de 0,075% d'acide acétique, teneur en furfural > 50 ppm et < 0,05%, teneur en benzaldéhyde > 50 ppm et < 0,05%, teneur en protoanémonine > 50 ppm et < 0,05%.

### FIGURES

La figure 1 représente de manière schématique un mode de réalisation d'une installation selon l'invention.
La figure 2 est un diagramme représentant le nombre d'étages théoriques à reflux total en fonction du trafic hydraulique dans une colonne à distiller un mélange binaire acide acétique - acide propénoïque.

### DESCRIPTION DETAILLEE

L'invention est maintenant décrite plus en détail et de façon non limitative dans la description qui suit.

Dans la présente invention, le terme «(méth)acrylique» signifie «acrylique» ou «méthacrylique». Par souci de simplification, la suite de l'exposé fera référence à la production d'acide acrylique, mais s'applique également par analogie à la production d'acide méthacrylique.

Le terme « solvant organique externe » désigne tout composé organique dans lequel l'acide (méth)acrylique est soluble et dont l'origine est extérieure au procédé, utilisé comme solvant d'absorption, d'extraction ou de distillation azéotropique.

Le terme « solvant azéotropique » désigne tout solvant organique présentant la propriété de former un mélange azéotropique avec l'eau.

Le terme « non condensable » ou « incondensable » désigne les composés dont le point d'ébullition est inférieur à la température de 20°C sous la pression atmosphérique.

Le terme « léger », qualifiant les composés sous-produits, désigne les composés dont le point d'ébullition est inférieur à celui de l'acide (méth)acrylique sous la pression de travail considérée, et par analogie, le terme « lourd » désigne les composés dont le point d'ébullition est supérieur à celui de l'acide (méth)acrylique.

Tous les pourcentages et teneurs indiqués sont massiques.

Le terme « acide acrylique technique » correspond à une solution d'acide acrylique de pureté supérieure à 98,5% et contenant moins de 0,3% en eau, moins de 0,075% d'acide acétique, teneur en furfural > 50 ppm et < 0,05%, teneur en benzaldéhyde > 50 ppm et < 0,05%, teneur en protoanémonine > 50 ppm et < 0,05%. Le terme « technique » indique ici que l'acide (méth)acrylique répond à des critères de très haute qualité permettant son utilisation dans la fabrication d'ester sans autre traitement de purification. Le procédé selon l'invention permet de garantir une qualité d'acide acrylique technique toujours proche de 99,5%, ce qui permet de diminuer la quantité d'acide acrylique technique à mettre en œuvre dans des procédés de fabrication d'ester. A titre d'exemple, pour une installation produisant 100.000 T/an d'acrylate de 2-éthylehexyle la quantité massique d'acide acrylique 100% de pureté est de 39000 T/an. Augmenter la qualité de l'acide acrylique technique de 98,5% à 99,5% permet d'engager 400 T d'acide acrylique technique en moins dans le procédé et diminuer ainsi de 400 T la quantité de sous-produits à traiter.

Cet acide acrylique technique, peut être utilisé, sans autre purification, pour produire des esters. Cette qualité « technique » ne va cependant pas être suffisante lorsque cet acide acrylique est destiné à la fabrication de polymères. Dans ce cas il sera nécessaire d'utiliser de « l'acide acrylique dit glacial » qui est défini comme suit : ayant une pureté supérieure à 99,7% et des teneurs en impuretés comme suit : teneur en furfural < 2 ppm ; teneur en aldéhydes totaux (furfural, benzaldéhyde, acroléine) < 10 ppm, de préférence < 4 ppm ; teneur en protoanémonine < 2 ppm.

La présente invention concerne un procédé de purification d'acide (méth)acrylique en l'absence de solvant organique et en l'absence d'un traitement chimique des aldéhydes, à partir d'un mélange réactionnel gazeux comprenant de l'acide (méth)acrylique obtenu par oxydation en phase gazeuse d'un précurseur de l'acide (méth)acrylique, ledit procédé comprenant les étapes suivantes :
a) soumettre ledit mélange réactionnel à une déshydratation, sans utiliser de solvant azéotropique, dans une colonne de déshydratation, conduisant à l'obtention d'un flux de tête et un flux de pied,
b) envoyer au moins une partie dudit flux de pied de la colonne de déshydratation dans la partie supérieure d'une colonne de finition, où on procède à une distillation des produits légers, à l'élimination des produits lourds en pied de cette colonne et au soutirage d'un flux latéral comprenant de l'acide acrylique technique en phase liquide,
c) envoyer ledit flux latéral de la colonne de finition au tiers inférieur d'une troisième colonne de distillation, conduisant à l'obtention d'un flux de tête, un flux latéral et un flux de pied, ledit flux de tête chargé en acide acrylique glacial étant soumis à une condensation puis renvoyé après condensation, en partie dans cette colonne sous forme de reflux, et en partie étant récupéré, ledit flux latéral chargé en acide acrylique technique étant soutiré dans la moitié du bas de ladite colonne de distillation.

Selon un mode de réalisation, au moins une partie du flux de tête de la colonne de déshydratation est soumise à une condensation, puis est renvoyée dans la colonne de déshydratation sous forme de reflux.

Selon un mode de réalisation, au moins une partie du flux de pied de la colonne de déshydratation est renvoyée sous forme de reflux dans la partie inférieure de cette colonne pour former une boucle de recirculation.

Selon un mode de réalisation, lesdits produits légers, composés d'eau et d'acide acétique, sont extraits en tête de la colonne de finition, puis soumis à une condensation. Au moins une partie du condensat ainsi obtenu est renvoyée vers la colonne de déshydratation pour être mélangée au flux de ladite boucle de recirculation. Selon un mode de réalisation, au moins une partie dudit condensat est recyclée comme reflux liquide à la tête de la colonne de finition.

Selon un mode de réalisation, le flux résiduel récupéré en pied de la colonne de distillation est recyclé vers un atelier d'estérification fabriquant des esters (méth)acryliques en C₁-C₈, sans purification supplémentaire.

Selon un mode de réalisation, le flux latéral soutiré de la colonne de finition comprend une solution contenant au moins 98,5% acide acrylique, moins de 0,3% en eau, moins de 0,075% d'acide acétique, et ayant une teneur en furfural supérieure à 50 ppm et inférieure à 0,05%, une teneur en benzaldéhyde supérieure à 50 ppm et inférieure à 0,05%, et une teneur en protoanémonine supérieure à 50 ppm et inférieure à 0,05%.

Selon un mode de réalisation, ledit flux de tête de la colonne de distillation comprend, après condensation, une solution contenant au moins 99,7% acide acrylique et des teneurs en impuretés comme suit : teneur en furfural inférieure à 2 ppm, une teneur en aldéhydes totaux (furfural, benzaldéhyde, acroléine) inférieure à 10 ppm, de préférence à 4 ppm, et une teneur en protoanémonine inférieure à 2 ppm.

Selon un mode de réalisation, le mélange réactionnel gazeux à purifier comporte un rapport massique eau/acide acrylique compris entre 0,3 et 2, de préférence entre 0,3 et 1,2. Ce mélange réactionnel comprend, en plus de l'eau et l'acide acrylique, des produits légers incondensables tels que l'azote, l'oxygène, le monoxyde et le dioxyde de carbone, ainsi que différents sous-produits légers ou lourds de différente nature chimique, pouvant être des aldéhydes légers comme l'acroléine, le formaldéhyde, l'acétaldéhyde ou le glyoxal, des aldéhydes lourds tels que le furfuraldéhyde ou le benzaldéhyde, des acides légers tels que l'acide formique, l'acide acétique ou l'acide propionique, des acides lourds tels que l'acide maléique, l'acide benzoïque ou l'acide 2-butènoïque, et de la protoanémonine composé lourd de type lactone.

Selon un mode de réalisation, le flux de pied de la colonne de déshydratation comprend essentiellement de l'acide acrylique (84-90%), de l'acide acétique (2-10%), de l'eau (2-10%), et des sous-produits lourds.

Selon un mode de réalisation, la colonne de déshydratation comprend de 5 à 50 plateaux théoriques, de préférence de 20 à 30 plateaux théoriques.

Avantageusement, la colonne de déshydratation fonctionne à la pression atmosphérique ou légèrement supérieure, jusqu'à une pression absolue de 1,5×10⁵ Pa.

Avantageusement, la température dans la partie supérieure de la colonne de déshydratation est d'au moins 40°C, de préférence est comprise entre 40°C et 80°C. La température du flux de pied de la colonne de déshydratation ne dépasse pas de préférence 120°C.

Selon un mode de réalisation, la colonne de finition est une colonne de distillation classique comprenant de 5 à 30 plateaux théoriques, de préférence de 8 à 20 plateaux théoriques. La colonne de finition fonctionne à une pression inférieure à la pression atmosphérique, permettant de fonctionner à des températures relativement faibles, évitant ainsi la polymérisation des produits insaturés présents, et minimisant la formation de sous-produits lourds.

Avantageusement, la colonne de finition fonctionne sous une pression absolue allant de 5 kPa à environ 60 kPa, la température du flux de tête étant avantageusement comprise entre 40°C et environ 90°C, et la température du flux de pied étant comprise entre 60°C et 120°C.

Selon un mode de réalisation, la colonne de distillation est une colonne de distillation classique comprenant de 15 à 30 plateaux théoriques, de préférence de 20-25 plateaux théoriques. Cette colonne fonctionne à une pression inférieure à la pression atmosphérique, permettant de fonctionner à des températures relativement faibles, évitant ainsi la polymérisation des produits insaturés présents, et minimisant la formation de sous-produits lourds.

Selon un mode de réalisation, des inhibiteurs de polymérisation, préférentiellement l'éther méthylique de l'hydroquinone (EMHQ), acétate de manganèse, hydroquinone, phénothiazine, ou leurs mélanges, en quantité adéquate, sont injectés en tête de toutes les colonnes pour protéger le flux condensé contre une polymérisation au niveau du condenseur, dans le bac de stockage et lors du transport avant utilisation de l'acide acrylique, en répondant aux exigences de réactivité en polymérisation.

Selon un mode de réalisation, au moins un inhibiteur est également injecté en amont de chaque condenseur, de façon à empêcher la formation de polymère lors de la condensation du mélange gazeux distillé et dans la colonne, grâce à la présence de cet inhibiteur dans le reflux liquide renvoyé en tête de colonne.

Selon un mode de réalisation, de l'air ou de l'air appauvri est injecté en bas des colonnes de finition et de distillation, de préférence dans une proportion volumique de 0,1 à 0,5% d'oxygène par rapport au débit total d'AA distillé.

Les sections de la colonne de distillation sont équipées de plateaux à contre-courant caractérisés par l'absence de trop pleins ou déversoirs tels: les plateaux Dual Flow, Turbo grid, Ripple Trays ou à clapets sans déversoirs. Ces plateaux à contre-courant fonctionnent de la façon décrite par JP. Wauquier, Institut Français du Pétrole, Le Raffinage du Pétrole, 1998, tome 2 : Procédés de séparation, chapitre 5, p 287. Le liquide et le gaz passent alternativement à travers les orifices, ce qui crée le caractère auto-nettoyant. Ces plateaux peuvent être efficaces pour effectuer une séparation lorsqu'ils fonctionnent dans leur condition de design, avec cependant une flexibilité de fonctionnement sévèrement limité car :
- à faible débit gazeux le liquide tend à passer à travers les orifices rapidement sans séjourner à la surface du plateau, réduisant ainsi le temps de contact entre les phases et par conséquent l'efficacité des plateaux ;
- à fort débit gazeux, le liquide sur le plateau est projeté, ne peut plus s'écouler à travers les orifices entraînant un phénomène d'engorgement.

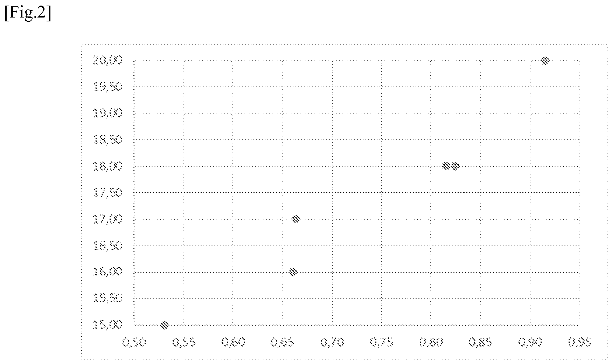

L'article de J.A. Garcia et J.R. Fair dans Ind.Eng. Res., 2002, 41, 632-1640 ou la Figure 2 annexée illustrent bien la perte d'efficacité de la colonne en fonction de l'hydraulique de la colonne et par la même de la charge de la colonne. Ainsi, lorsque le débit de gaz est diminué par 2, l'efficacité d'un plateau peut décroitre de 80% à 40% (voir la figure 3 de cette publication).

On comprend bien qu'avec une telle variation d'efficacité de séparation, le design d'une installation permettant d'obtenir des proportions variables d'acide acrylique technique et d'acide acrylique glacial s'avère problématique et la présente invention entend proposer une solution à cette problématique.

Avantageusement, la colonne de distillation fonctionne sous une pression absolue allant de 5 kPa à environ 60 kPa, la température du flux de tête étant avantageusement comprise entre 40°C et environ 90°C, et la température du flux de pied étant comprise entre 60°C et 120°C.

Pour la colonne de distillation, le taux de reflux, que l'on peut définir comme le débit de recyclage de la tête de colonne vers la colonne, rapporté à celui de soutirage latéral, est compris entre 1,5 et 4, de préférence entre 2 et 3, par exemple est égal à 2,5. Dans ces conditions, il est possible d'obtenir un bon compromis entre la taille de colonne et le nombre d'étages de séparation à utiliser et l'énergie à mettre en œuvre pour assurer cette séparation.

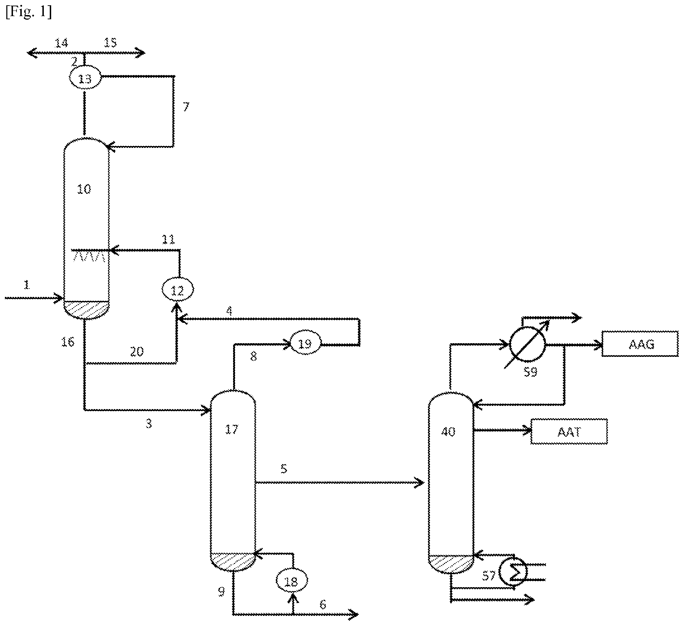

Selon le mode de réalisation du procédé représenté à la Figure 1, un mélange réactionnel gazeux 1 comprenant de l'acide acrylique obtenu par oxydation en phase gazeuse d'un précurseur de l'acide acrylique alimente une première colonne de distillation 10. Le mélange réactionnel gazeux peut être préalablement refroidi avant d'être soumis à une déshydratation dans la colonne de déshydratation 10.

La colonne de déshydratation conduit à un flux de tête 2 dont au moins une partie est condensée dans un condenseur 13 et renvoyée à la colonne de déshydratation sous forme de reflux 7 pour absorber l'acide acrylique, l'autre partie (flux 14 et 15) comprenant les composés légers incondensables étant généralement envoyée partiellement ou totalement à un dispositif d' épuration ou recyclée en partie vers d'autres étapes du procédé de production d'acide acrylique, de préférence dans une étape située en amont du réacteur de production du mélange réactionnel 1.

La totalité du flux de tête de la colonne de déshydratation peut être envoyée dans le condenseur de tête 13.

L'étape de déshydratation a pour but d'éliminer dans un flux de tête l'essentiel de l'eau présente dans le mélange réactionnel, mais aussi les composés légers incondensables et les composés légers condensables. Elle génère un flux de tête 2 comprenant l'essentiel de l'eau et des composés légers, avec de l'acide acrylique et des composés lourds en quantité très faible, et un flux de pied 16 appauvri en composés légers comprenant la quasi-totalité de l'acide acrylique avec des sous-produits lourds, et une teneur massique en eau généralement inférieure à 10%, de préférence inférieure à 7%.

Le flux de pied 16 de la colonne de déshydratation est envoyé au moins en partie (flux 3), en tête d'une seconde colonne de distillation 17, dite colonne de finition, dans laquelle sont séparés un flux de tête 8 et un flux de pied 9.

Une partie 20 du flux liquide 16 de pied de la colonne de déshydratation est envoyée dans un échangeur de chaleur 12 qui peut être un réchauffeur ou un refroidisseur et réinjectée dans la colonne de déshydratation, de façon à constituer une boucle de recirculation en pied. De préférence, la partie 11 de la boucle de pied est réinjectée entre l'alimentation du mélange gazeux réactionnel et la tête de colonne de déshydratation.

Le reste (flux 3) du flux liquide 16 est envoyé en alimentation de la colonne de finition 17. Cette colonne de distillation est associée en pied à au moins un rebouilleur 18 et en tête à un condenseur 19.

Le flux gazeux de tête 8 de la colonne de finition est envoyé dans le condenseur 19, et le flux liquide sortant 4 est renvoyé vers la colonne de déshydratation, mélangé au flux de la boucle de pied de la colonne de déshydratation. Le flux de tête 8 comprend essentiellement de l'eau et les sous-produits légers condensables.

Le flux 9 séparé en pied de la colonne de finition comprend l'essentiel des sous-produits lourds, notamment des produits d'addition de Michael tels que l'acide 3-acryloxypropionique, de l'anhydride/acide maléique, de l'acide benzoïque, le dimère de l'acide acrylique ainsi que des inhibiteurs de polymérisation. Ce flux 9 peut être en partie recyclé dans le bas de la colonne de finition, ou utilisé comme matière première des esters acryliques après élimination des dimères de l'acide acrylique.

Un flux 5 comprenant de l'acide acrylique purifié sous forme de liquide ou de vapeur, est extrait de la colonne de finition par soutirage latéral. Ce flux 5 correspond à de l'acide acrylique technique.

Ce flux 5 après condensation est envoyé vers une troisième colonne de distillation pour obtenir l'acide acrylique glacial et l'acide acrylique technique. Dans cette configuration, l'ensemble de l'acide acrylique technique issu de la colonne de finition alimente la colonne d'obtention de l'acide acrylique glacial assurant ainsi une charge hydraulique d'alimentation constante de cette troisième colonne.

Cette unité de distillation (40) comporte, en particulier après condensation, un reflux en tête de la colonne de distillation, un soutirage des produits lourds en pied de colonne, un soutirage liquide d'acide acrylique glacial en tête de colonne (AAg) et une phase gazeuse collectée vers l'évent de l'unité et un soutirage latéral pour l'acide acrylique technique.

Cette colonne de distillation est associée en pied à au moins un rebouilleur 57 et en tête à un condenseur 59.

La colonne 40 est alimentée dans le premier tiers du bas.

L'acide acrylique glacial après condensation est soutirée en tête de colonne. Les incondensables (air, organiques non condensés) sont envoyés dans le réseau d'évent du procédé. L'acide acrylique technique est soutiré au moins 1 plateau en dessous du soutirage d'acide acrylique glacial, de préférence dans la première moitié du bas de colonne voire dans le premier tiers du bas de colonne. Ce soutirage latéral peut être effectué en phase gazeuse ou liquide de préférence liquide.

Le pied de la colonne peut avantageusement être re-mélangé avec cet acide acrylique technique tout en obtenant un produit ayant une pureté supérieure à 99,5%.

Le flux gazeux de tête de la colonne 40 est envoyé dans le condenseur 59, et le flux liquide sortant est de l'acide acrylique glacial ayant une pureté supérieure à 99,7% et des teneurs massiques en impuretés comme suit : teneur en furfural < 2 ppm ; teneur en aldéhydes totaux (furfural, benzaldéhyde, acroléine) < 10 ppm, de préférence < 4 ppm ; teneur en protoanémonine < 2 ppm.

Le rapport massique entre le flux soutiré en pied et le flux d'alimentation est compris entre 1% et 40% de préférence entre 5% et 10%.

Le flux récupéré en pied de l'unité de distillation 40 est avantageusement re-mélangé au flux d'acide acrylique technique vers une unité d'estérification sans traitement supplémentaire avec une pureté d'au moins 99,5%.

En tête du condenseur 59, le flux gazeux résiduel est envoyé dans le circuit d'évent du procédé.

L'invention sera maintenant illustrée par les exemples suivants, qui n'ont pas pour but de limiter la portée de l'invention, définie par les revendications annexées.

### EXEMPLES

Les exemples suivants illustrent l'invention sans la limiter.

Dans les exemples, les pourcentages sont indiqués en poids pour les composés principaux sauf indication contraire, et les abréviations suivantes ont été utilisées :
H20 : eau
ACRA : acide acrylique
Benzal : benzaldéhyde
HAC : acide acétique
FURF : furfuraldéhyde
DIMR : dimères de l'acide acrylique

### Acide Propénoïque

La Protoanémonine a été assimilée dans nos simulations ASPEN au benzaldéhyde.

Exemple 1. Efficacité de séparation d'un mélange binaire acide acétique - acide propénoïque dans une colonne à distiller diamètre 300 mm comportant 50 plateaux Dual Flow à reflux total à une pression de tête de 90 mm Hg (0.012 MPa).

L'analyse des compositions de tête et de pied par chromatographie en phase gazeuse à différents pourcentages d'engorgement permet, après utilisation du logiciel Aspen, de déterminer le nombre d'étages théoriques à reflux total en fonction du trafic hydraulique dans la colonne.

Comme le montre le graphique de la Fig. 2 annexée, l'efficacité de séparation que l'on exprime ici en termes de nombre d'étages théoriques est fortement dépendante de l'hydraulique de la colonne.

**Exemple 2 (****figure 1**) :Purification d'un mélange réactionnel gazeux comprenant de l'acide acrylique obtenu par oxydation en phase gazeuse d'un précurseur de l'acide acrylique, au moyen de trois colonnes de distillation.

Le procédé de récupération d'acide acrylique technique et d'acide acrylique glacial représenté sur la Figure 1, fournit en soutirage latéral de la colonne de finition (17), un flux (5) d'acide acrylique purifié.

Le flux (3) de pied de la colonne de déshydratation (10) constitue l'alimentation au niveau du plateau supérieur de la colonne de finition (17). La colonne de finition (17) comporte 17 étages théoriques, et le soutirage latéral est réalisé au niveau du plateau théorique 16 à compter à partir du haut de la colonne.

Le flux (5) alimente après condensation et ajout d'une solution de stabilisation la colonne (40) comportant 24 étages théoriques au plateau 20. L'acide acrylique glacial est soutiré en tête de colonne après condensation et retour partiel d'une partie du flux dans la colonne pour assurer le reflux de celle-ci. L'acide acrylique en pied de la colonne peut être re-mélangé avec l'acide acrylique technique obtenu au soutirage latéral. La colonne est également stabilisée au condenseur et au reflux et de l'air est injecté en pied de colonne (non représenté.). L'acide acrylique technique à raison de 50% du flux d'alimentation de la colonne est soutiré au plateau 16.

Le tableau 1 ci-après présente les différentes qualités de flux.

**[Tableau 1]**

| FLUX | | **3** | **8** | **6** | **Alim C 40** | **AAG** | **AAT** | **Pied C40** |
|---|---|---|---|---|---|---|---|---|
| Température | C | 69,6 | 72,9 | 30,0 | 20,1 | 20,0 | 93,2 | 95,3 |
| Pression | bar | 3,2 | 0,1 | 0,2 | 1,0 | 0,1 | 0,2 | 0,2 |
| Débit mass. | kg/h | 188,2 | 121,4 | 6,5 | 65,4 | 32,0 | 32,7 | 4,0 |
| Mass | | | | | | | | |
| Fractions | | | | | | | | |
| H₂O | | 5,32% | 8,25% | 0,00% | 0,01% | 0,01% | 0,00% | 0,00% |
| HAC | | 11,00% | 17,04% | 0,01% | 0,05% | 0,09% | 0,01% | 0,00% |
| ACRA | | 82,97% | 73,84% | 82,73% | 99,76% | 99,85% | 99,86% | 97,58% |
| FURF | | 0,01% | 0,01% | 0,06% | 0,02% | 0,000088% | 0,028129% | 0,16% |
| BENZALD | | 0,02% | 0,01% | 0,15% | 0,03% | 0,000000% | 0,021777% | 0,34% |
| DIMR | | 0,24% | 0,00% | 6,80% | 0,00% | 0,000000% | 0,001142% | 0,05% |

Avec ce procédé on obtient de l'acide acrylique technique de très grande qualité ce qui permet de valoriser également le pied de la C40 par mélange. Dans ce cas la pureté est de 99,57%.

Avec moins de 1 ppm de furfural et sans benzaldéhyde l'acide acrylique glacial est conforme aux spécifications.

L'énergie mise en œuvre pour la colonne de finition et d'obtention de l'acide acrylique glacial est de 40043 kcal/h.

## Revendications

1. Procédé de purification d'acide (méth)acrylique en l'absence de solvant organique et en l'absence d'un traitement chimique des aldéhydes, à partir d'un mélange réactionnel gazeux comprenant de l'acide (méth)acrylique obtenu par oxydation en phase gazeuse d'un précurseur de l'acide (méth)acrylique, ledit procédé comprenant les étapes suivantes :
- soumettre ledit mélange réactionnel à une déshydratation, sans utiliser de solvant azéotropique, dans une colonne de déshydratation, conduisant à l'obtention d'un flux de tête et un flux de pied,
- envoyer au moins une partie dudit flux de pied de la colonne de déshydratation dans la partie supérieure d'une colonne de finition, où on procède à une distillation des produits légers, à l'élimination des produits lourds en pied de cette colonne et au soutirage d'un flux latéral,
- envoyer ledit flux latéral de la colonne de finition au tiers inférieur d'une troisième colonne de distillation, conduisant à l'obtention d'un flux de tête, un flux latéral et un flux de pied, ledit flux de tête étant soumis à une condensation puis renvoyé après condensation, en partie dans cette colonne sous forme de reflux, et en partie étant récupéré, ledit flux latéral étant soutiré dans la moitié du bas de ladite colonne de distillation.

2. Procédé selon la revendication 1, dans lequel au moins une partie du flux de tête de la colonne de déshydratation est soumise à une condensation, puis est renvoyée dans la colonne de déshydratation sous forme de reflux.

3. Procédé selon l'une des revendications 1 et 2, dans lequel au moins une partie du flux de pied de la colonne de déshydratation est renvoyée sous forme de reflux dans la partie inférieure de cette colonne pour former une boucle de recirculation.

4. Procédé selon l'une quelconque des revendications précédentes, dans lequel lesdits produits légers, composés d'eau et d'acide acétique, extraits en tête de la colonne de finition, sont soumis à une condensation, au moins une partie du condensat ainsi obtenu étant renvoyée vers la colonne de déshydratation pour être mélangée au flux de ladite boucle de recirculation.

5. Procédé selon l'une quelconque des revendications précédentes, dans lequel le flux résiduel récupéré en pied de la colonne de distillation est recyclé vers un atelier d'estérification fabriquant des esters (méth)acryliques en C₁-C₈, sans purification supplémentaire.

6. Procédé selon l'une quelconque des revendications précédentes, dans lequel le flux latéral soutiré de la colonne de finition comprend une solution contenant au moins 98,5% acide acrylique, moins de 0,3% en eau, moins de 0,075% d'acide acétique, et ayant une teneur en furfural supérieure à 50 ppm et inférieure à 0,05%, une teneur en benzaldéhyde supérieure à 50 ppm et inférieure à 0,05%, et une teneur en protoanémonine supérieure à 50 ppm et inférieure à 0,05%.

7. Procédé selon l'une quelconque des revendications 1 à 6, dans lequel le flux de tête de la colonne de distillation comprend, après condensation, une solution contenant au moins 99,7% acide acrylique et des teneurs en impuretés comme suit : teneur en furfural inférieure à 2 ppm, une teneur en aldéhydes totaux (furfural, benzaldéhyde, acroléine) inférieure à 10 ppm, de préférence à 4 ppm, et une teneur en protoanémonine inférieure à 2 ppm.

8. Procédé selon l'une quelconque des revendications 1 à 6, dans lequel le flux de soutirage latéral comprend, après condensation, une solution contenant au moins 99,7% d'acide acrylique, moins de 0,3% d'eau, une teneur en furfural supérieure à 50 ppm, une teneur en benzaldéhyde supérieure à 50 ppm et une teneur en protoanémonine supérieure à 50 ppm.

9. Procédé selon l'une quelconque des revendications 1 à 6, dans lequel le flux de pied contenant au moins 97% d'acide acrylique est mélangé avec le flux d'acide acrylique obtenu au soutirage latéral ou recyclé vers une unité d'estérification sans traitement supplémentaire.

10. Procédé selon l'une quelconque des revendications précédentes, dans lequel la colonne de déshydratation comprend de 5 à 50 plateaux théoriques, de préférence de 20 à 30 plateaux théoriques et fonctionne à la pression atmosphérique ou légèrement supérieure, jusqu'à une pression absolue de 1,5×10⁵ Pa.

11. Procédé selon l'une quelconque des revendications précédentes, dans lequel la température dans la partie supérieure de la colonne de déshydratation est d'au moins 40°C, de préférence comprise entre 40°C et 80°C, et la température du flux de pied de la colonne de déshydratation est inférieure à 120°C.

12. Procédé selon l'une quelconque des revendications précédentes, dans lequel la colonne de finition comprend de 5 à 30 plateaux théoriques, de préférence de 8 à 20 plateaux théoriques, et fonctionne sous une pression absolue allant de 5 kPa à 60 kPa, la température du flux de tête étant comprise entre 40°C et environ 90°C, et la température du flux de pied étant comprise entre 60°C et 120°C.

13. Procédé selon l'une quelconque des revendications précédentes, dans lequel la colonne de distillation fonctionne sous une pression absolue allant de 5 kPa à environ 60 kPa, la température du flux de tête étant avantageusement comprise entre 40°C et environ 90°C, et la température du flux de pied étant comprise entre 60°C et 120°C.

14. Procédé selon l'une quelconque des revendications précédentes, dans lequel le taux de reflux de la colonne de distillation, défini comme le débit de recyclage de la tête vers la colonne, rapporté à celui du soutirage d'acide (meth)acrylique en tête de colonne, est compris entre 1,5 et 4, de préférence entre 2 et 3.

15. Procédé selon l'une quelconque des revendications précédentes, dans lequel un inhibiteur de polymérisation choisi parmi l'éther méthylique de l'hydroquinone, acétate de manganèse, hydroquinone, phénothiazine et leurs mélanges sont injectés en tête de toutes les colonnes.

16. Procédé selon l'une quelconque des revendications précédentes, dans lequel le ratio d'obtention d'acide acrylique glacial / acide acrylique technique varie de 10 à 90%, de préférence de 30 à 70% .

## Patentansprüche

1. Verfahren zur Reinigung von (Meth)Acrylsäure ohne organisches Lösungsmittel und ohne eine chemische Behandlung der Aldehyde ausgehend von einem gasförmigen Reaktionsgemisch, das durch Gasphasenoxidation eines Vorläufers der (Meth)Acrylsäure erhaltene (Meth)Acrylsäure umfasst, wobei das Verfahren die folgenden Schritte umfasst:
- Unterziehen des Reaktionsgemisches einer Dehydratisierung, ohne azeotropes Lösungsmittel zu verwenden, in einer Dehydratisierungskolonne, was zum Erhalten eines Kopfstroms und eines Sumpfstroms führt,
- Leiten mindestens eines Teils des Sumpfstroms der Dehydratisierungskolonne in den oberen Teil einer Endbehandlungskolonne, wo eine Destillation der leichtflüchtigen Stoffe, die Eliminierung der schwerflüchtigen Stoffe am Sumpf dieser Kolonne und das Abziehen eines Seitenstroms vorgenommen werden,
- Leiten des Seitenstroms der Endbehandlungskolonne zum unteren Drittel einer dritten Destillationskolonne, was zum Erhalten eines Kopfstroms, eines Seitenstroms und eines Sumpfstroms führt, wobei der Kopfstrom einer Kondensation unterzogen wird, dann nach Kondensation zum Teil in Form eines Rücklaufs in diese Kolonne zurückgeleitet wird und zum Teil zurückgewonnen wird, wobei der Seitenstrom in der Hälfte des Sumpfs der Destillationskolonne abgezogen wird.

2. Verfahren nach Anspruch 1, wobei mindestens ein Teil des Kopfstroms der Dehydratisierungskolonne einer Kondensation unterzogen wird, dann als Rücklauf in die Dehydratisierungskolonne zurückgeleitet wird.

3. Verfahren nach einem der Ansprüche 1 und 2, wobei mindestens ein Teil des Sumpfstroms der Dehydratisierungskolonne in Form eines Rücklaufs in den unteren Teil dieser Kolonne zurückgeleitet wird, um eine Kreislaufschleife zu bilden.

4. Verfahren nach einem der vorhergehenden Ansprüche, wobei die aus Wasser und Essigsäure zusammengesetzten leichtflüchtigen Stoffe, die am Kopf der Endbehandlungskolonne entnommen werden, einer Kondensation unterzogen werden, wobei mindestens ein Teil des so erhaltenen Kondensats zu der Dehydratisierungskolonne zurückgeleitet wird, um mit dem Strom der Kreislaufschleife gemischt zu werden.

5. Verfahren nach einem der vorhergehenden Ansprüche, wobei der am Sumpf der Destillationskolonne zurückgewonnene Reststrom ohne zusätzliche Reinigung zu einer Veresterungsanlage, die C₁-C₈- (Meth)Acrylester herstellt, zurückgeführt wird.

6. Verfahren nach einem der vorhergehenden Ansprüche, wobei der aus der Endbehandlungskolonne abgezogene Seitenstrom eine Lösung umfasst, die mindestens 98,5 % Acrylsäure, weniger als 0,3 % Wasser, weniger als 0,075 % Essigsäure enthält und einen Furfuralgehalt von mehr als 50 ppm und weniger als 0,05 %, einen Benzaldehydgehalt von mehr als 50 ppm und weniger als 0,05 % und einen Protoanemoningehalt von mehr als 50 ppm und weniger als 0,05 % hat.

7. Verfahren nach einem der Ansprüche 1 bis 6, wobei der Kopfstrom der Destillationskolonne, nach Kondensation, eine Lösung umfasst, die mindestens 99,7 % Acrylsäure und Gehalte an Verunreinigungen wie folgt enthält: Furfuralgehalt unter 2 ppm, einen Gehalt an Gesamtaldehyden (Furfural, Benzaldehyd, Acrolein) unter 10 ppm, bevorzugt unter 4 ppm, und einen Protoanemoningehalt unter 2 ppm.

8. Verfahren nach einem der Ansprüche 1 bis 6, wobei der Seitenabzugsstrom, nach Kondensation, eine Lösung umfasst, die mindestens 99,7 % Acrylsäure, weniger als 0,3 % Wasser, einen Furfuralgehalt über 50 ppm, einen Benzaldehydgehalt über 50 ppm und einen Protoanemoningehalt über 50 ppm enthält.

9. Verfahren nach einem der Ansprüche 1 bis 6, wobei der Sumpfstrom, der mindestens 97 % Acrylsäure enthält, mit dem am Seitenabzug erhaltenen Acrylsäurestrom gemischt wird oder ohne zusätzliche Behandlung zu einer Veresterungseinheit zurückgeführt wird.

10. Verfahren nach einem der vorhergehenden Ansprüche, wobei die Dehydratisierungskolonne 5 bis 50 theoretische Böden, bevorzugt 20 bis 30 theoretische Böden umfasst und bei atmosphärischem oder geringfügig höherem Druck bis zu einem absoluten Druck von 1,5×10⁵ Pa betrieben wird.

11. Verfahren nach einem der vorhergehenden Ansprüche, wobei die Temperatur in dem oberen Teil der Dehydratisierungskolonne mindestens 40 °C, bevorzugt zwischen 40 °C und 80 °C beträgt und die Temperatur des Sumpfstroms der Dehydratisierungskolonne weniger als 120 °C beträgt.

12. Verfahren nach einem der vorhergehenden Ansprüche, wobei die Endbehandlungskolonne 5 bis 30 theoretische Böden, bevorzugt 8 bis 20 theoretische Böden umfasst und bei einem absoluten Druck von 5 kPa bis 60 kPa betrieben wird, wobei die Temperatur des Kopfstroms zwischen 40 °C und etwa 90 °C beträgt und wobei die Temperatur des Sumpfstroms zwischen 60 °C und 120 °C beträgt.

13. Verfahren nach einem der vorhergehenden Ansprüche, wobei die Destillationskolonne und unter einem absoluten Druck betrieben wird, der von 5 kPa bis etwa 60 kPa reicht, wobei die Temperatur des Kopfstroms zwischen 40 °C und etwa 90 °C beträgt und wobei die Temperatur des Sumpfstroms zwischen 60 °C und 120 °C beträgt.

14. Verfahren nach einem der vorhergehenden Ansprüche, wobei das Rücklaufverhältnis der Destillationskolonne, definiert als der Rückführungsvolumenstrom vom Kopf zur Kolonne bezogen auf den des Abzugs von (Meth)Acrylsäure am Kolonnenkopf, zwischen 1,5 und 4, bevorzugt zwischen 2 und 3 beträgt.

15. Verfahren nach einem der vorhergehenden Ansprüche, wobei ein Polymerisationshemmer, der aus dem Methylether von Hydrochinon, Manganacetat, Hydrochinon, Phenothiazin und ihren Mischungen ausgewählt ist, in den Kopf aller Kolonnen eingespritzt werden.

16. Verfahren nach einem der vorhergehenden Ansprüche, wobei das Verhältnis des Erhaltens von Eisacrylsäure/technischer Acrylsäure von 10 bis 90 %, bevorzugt von 30 bis 70 % variiert.

## Claims

1. A process for the purification of (meth)acrylic acid in the absence of organic solvent and in the absence of a chemical treatment of the aldehydes, from a gaseous reaction mixture comprising (meth)acrylic acid obtained by gas-phase oxidation of a precursor of (meth)acrylic acid, said process comprising the following stages:
- subjecting said reaction mixture to a dehydration, without using azeotropic solvent, in a dehydration column, resulting in a top stream and a bottom stream being obtained,
- sending at least a portion of said bottom stream from the dehydration column into the upper part of a finishing column, where the light products are distilled, the heavy products are removed at the bottom of this column and a side stream is withdrawn,
- sending said side stream from the finishing column to the lower third of a third distillation column, resulting in a top stream, a side stream and a bottom stream being obtained, said top stream being subjected to a condensation and then returned after condensation, partly in this column in reflux form and partly being recovered, said side stream being withdrawn in the half of the lower part of said distillation column.

2. The process as claimed in claim 1, in which at least a portion of the top stream from the dehydration column is subjected to a condensation, then is returned to the dehydration column in reflux form.

3. The process as claimed in either of claims 1 and 2, in which at least a portion of the bottom stream from the dehydration column is returned in reflux form to the lower part of this column to form a recirculation loop.

4. The process as claimed in any one of the preceding claims, in which said light products, composed of water and of acetic acid, extracted at the top of the finishing column, are subjected to a condensation, at least a portion of the condensate thus obtained being returned to the dehydration column to be mixed with the stream of said recirculation loop.

5. The process as claimed in any one of the preceding claims, in which the residual stream recovered at the bottom of the distillation column is recycled to an esterification plant manufacturing C₁-C₈ (meth)acrylic esters, without additional purification.

6. The process as claimed in any one of the preceding claims, in which the side stream withdrawn from the finishing column comprises a solution containing at least 98.5% of acrylic acid, less than 0.3% of water, less than 0.075% of acetic acid, and having a furfural content of greater than 50 ppm and less than 0.05%, a benzaldehyde content of greater than 50 ppm and less than 0.05%, and a protoanemonin content of greater than 50 ppm and less than 0.05%.

7. The process as claimed in any one of claims 1 to 6, in which the top stream from the distillation column comprises, after condensation, a solution containing at least 99.7% of acrylic acid and contents of impurities as follows: furfural content of less than 2 ppm, a content of total aldehydes (furfural, benzaldehyde, acrolein) of less than 10 ppm, preferably than 4 ppm, and a protoanemonin content of less than 2 ppm.

8. The process as claimed in any one of claims 1 to 6, in which the side stream withdrawal stream comprises, after condensation, a solution containing at least 99.7% of acrylic acid, less than 0.3% of water, a furfural content of greater than 50 ppm, a benzaldehyde content of greater than 50 ppm and a protoanemonin content of greater than 50 ppm.

9. The process as claimed in any one of claims 1 to 6, in which the bottom stream containing at least 97% of acrylic acid is mixed with the acrylic acid stream obtained in side stream withdrawal or recycled to an esterification unit without additional treatment.

10. The process as claimed in any one of the preceding claims, in which the dehydration column comprises from 5 to 50 theoretical plates, preferably from 20 to 30 theoretical plates, and operates at atmospheric pressure or slightly higher, up to an absolute pressure of 1.5 × 10⁵ Pa.

11. The process as claimed in any one of the preceding claims, in which the temperature in the upper part of the dehydration column is at least 40°C, preferably between 40°C and 80°C, and the temperature of the bottom stream from the dehydration column is less than 120°C.

12. The process as claimed in any one of the preceding claims, in which the finishing column comprises from 5 to 30 theoretical plates, preferably from 8 to 20 theoretical plates, and operates under an absolute pressure ranging from 5 kPa to 60 kPa, the temperature of the top stream being between 40°C and approximately 90°C and the temperature of the bottom stream being between 60°C and 120°C.

13. The process as claimed in any one of the preceding claims, in which the distillation column operates under an absolute pressure ranging from 5 kPa to approximately 60 kPa, the temperature of the top stream advantageously being between 40°C and approximately 90°C and the temperature of the bottom stream being between 60°C and 120°C.

14. The process as claimed in any one of the preceding claims, in which the reflux ratio of the distillation column, defined as the flow rate of recycling from the top to the column, with respect to that of the withdrawal of (meth)acrylic acid at the column top, is between 1.5 and 4, preferably between 2 and 3.

15. The process as claimed in any one of the preceding claims, in which a polymerization inhibitor chosen from hydroquinone methyl ether, manganese acetate, hydroquinone, phenothiazine and their mixtures are injected at the top of all the columns.

16. The process as claimed in any one of the preceding claims, in which the ratio for obtaining glacial acrylic acid/technical acrylic acid varies from 10% to 90%, preferably from 30% to 70%.
